# EUROPÄISCHE PATENTANMELDUNG

(11) **EP 4 417 859 A1**
(43) Veröffentlichungstag der Anmeldung: **21.08.2024**
(21) Anmeldenummer: 23156876.7
(22) Anmeldetag: 15.02.2023
(51) Int. Cl.: F16M 11/10, F16M 11/20, F16M 13/02

(54) **FEDERARMVORRICHTUNG UND TRAGARMSYSTEM ZUM HALTEN EINES MEDIZINISCHEN GERÄTS UND/ODER EINES BELEUCHTUNGSGERÄTS**

(71) Anmelder: Ondal Medical Systems GmbH, 36088 Hünfeld (DE)
(72) Erfinder: Abel, Markus, 36037 Fulda (DE)
(74) Vertreter: Graf von Stosch Patentanwaltsgesellschaft mbH

(57) **Zusammenfassung**

Die vorliegende Erfindung betrifft eine Federarmvorrichtung (1) für ein Tragarmsystem (100) zum Halten eines medizinischen Geräts und/oder eines Beleuchtungsgeräts, umfassend ein um eine erste Drehachse (21) drehbar an einer Befestigungseinheit (5) angeordnetes Federrohr (3), eine zumindest teilweise im Inneren des Federrohres (3) angeordnete, in Längsrichtung (2) des Federrohres (3) relativ zum Federrohr (3) verschiebbare Federstange (4), und ein Hebelelement (6), das um eine parallel zur ersten Drehachse (21) orientierte zweite Drehachse (22) drehbar mit der Federstange (4) verbunden ist und auf seiner der Federstange (4) gegenüberliegenden Seite um eine von der ersten Drehachse (21) beabstandete, zur ersten Drehachse (21) parallel orientierte dritte Drehachse (23) drehbar an der Befestigungseinheit (5) angeordnet ist, wobei das Hebelelement (6) bezogen auf die Längsrichtung (2) des Federrohres (3) radial außerhalb des Federrohres (3) angeordnet ist; sie betrifft ferner ein eine solche Federarmvorrichtung (1) umfassendes Tragarmsystem (100) zum Halten eines medizinischen Geräts und/oder eines Beleuchtungsgeräts.

## Beschreibung

### Technisches Gebiet

Die vorliegende Erfindung betrifft eine Federarmvorrichtung für ein Tragarmsystem zum Halten eines medizinischen Geräts und/oder eines Beleuchtungsgeräts und ein Tragarmsystem zum Halten eines medizinischen Geräts und/oder eines Beleuchtungsgeräts.

### Stand der Technik

Tragarmsysteme werden beispielsweise in Operationssälen zum örtlichen verlagerbaren Halten einer medizintechnischen Einrichtung bzw. eines medizintechnischen Geräts genutzt. Beispielsweise kann dazu ein Tragarm mittels einer Lageranordnung beweglich an einer Decke, einer Wand oder einem Boden verankert sein. Auch eine Befestigung auf einem Gestell mit Rollen ist möglich. Ein Tragarmsystem, welches zum Halten medizinischer Geräte ausgebildet ist, kann auch als medizinisches Tragsystem bezeichnet werden. Tragarmsystem weisen üblicherweise wenigstens eine Bewegungsachse auf, damit das gehaltene medizinischen Gerät, welches auch als medizintechnisches Gerät bezeichnet werden und beispielsweise ein Beleuchtungsgerät wie eine OP-Lampe, einen Monitor, Untersuchungsgeräte, einen oder mehrere Injektoren, OP-Besteck oder zahnmedizinische Bohrer aufweisen kann, gemäß jeweiliger Anforderungen bewegt werden kann. Die Lagerung, welche die Bewegung ermöglicht, kann auch als Gelenkvorrichtung oder Tragarmgelenkvorrichtung bezeichnet werden, wobei diese auch einen Tragarm umfassen kann. Damit kann das medizinische Gerät in eine angenehme und sichere Position zum Durchführen jeweiliger medizinischer Prozeduren und Überwachungen verbracht werden. Das örtliche Verlagern medizinischer Geräte ist bei vielen medizinischen Prozeduren essentiell, um dem medizinischen Personal die Arbeit zu erleichtern oder überhaupt zu ermöglichen.

Durch ein Schwenken eines Tragarms kann jedoch ein Hebelarm bezüglich dessen Lagerung verändert werden. Insbesondere bei einem Schwenken des Tragarms um eine im Wesentlichen horizontale Achse, wodurch die Höhe des daran gehaltenen medizinischen Geräts verändert werden kann, wird ein Hebelarm der Gewichtskraft des medizinischen Geräts gegenüber der Befestigung bzw. Lagerung des Tragarms verändert. Dennoch ist es gewünscht, dass das medizinische Gerät in einer durch das Schwenken des Tragarms eingestellten Position verbleibt und eine Verstellung der Position des medizinischen Geräts, insbesondere entgegen der Schwerkraft, ohne exzessiven Kraftaufwand für einen Bediener möglich sein soll.

Zu diesem Zweck werden häufig federbalancierte Tragarme, vorliegend als Federarme bzw. Federarmvorrichtungen bezeichnet, genutzt. Dabei werden zwei relativ zueinander bewegbare Armelemente vorgesehen, welche sich mittels einer Feder gegenseitig stützen. Durch Verschwenken des Federarms ändert sich der Hebelarm der Federkraft korrespondierend zu dem Hebelarm der Traglast, wodurch das medizinische Gerät in unterschiedlichen Positionen gleichermaßen gut gehalten werden kann. Alternativ oder zusätzlich kann auch die Feder durch das Schwenken komprimiert oder gestreckt werden, wodurch ebenfalls eine korrespondierende Stützkraft geändert werden kann.

Derartige Tragarmsysteme und Federarmvorrichtungen sind beispielsweise aus der EP 1 478 872 A1 zu entnehmen. Federarmvorrichtungen umfassen in der Regel ein um eine zumeist horizontale Drehachse drehbar an einer Befestigungseinheit angeordnetes Federrohr, eine zumindest teilweise im Inneren des Federrohres angeordnete, relativ zum Federrohr verschiebbare Federstange und ein Hebelelement, das an einem Ende im Inneren des Federrohres drehbar mit der Federstange verbunden ist und am anderen Ende drehbar an der Befestigungseinheit angeordnet ist.

### Darstellung der Erfindung

Ausgehend von dem bekannten Stand der Technik ist es eine Aufgabe der vorliegenden Erfindung, eine verbesserte Federarmvorrichtung für ein Tragarmsystem zum Halten eines medizinischen Geräts und/oder eines Beleuchtungsgeräts, sowie ein entsprechendes Tragarmsystem zum Halten eines medizinischen Geräts und/oder eines Beleuchtungsgeräts bereitzustellen. Insbesondere soll eine Federarmvorrichtung mit einem vergleichsweise großen Bewegungsumfang bereitgestellt werden.

Die Aufgabe wird durch eine Federarmvorrichtung für ein Tragarmsystem zum Halten eines medizinischen Geräts und/oder eines Beleuchtungsgeräts mit den Merkmalen des Anspruchs 1 gelöst. Vorteilhafte Weiterbildungen ergeben sich aus den Unteransprüchen, der Beschreibung und den Figuren.

Entsprechend wird eine Federarmvorrichtung für ein Tragarmsystem zum Halten eines medizinischen Geräts und/oder eines Beleuchtungsgeräts vorgeschlagen, umfassend ein um eine erste Drehachse drehbar an einer Befestigungseinheit angeordnetes Federrohr, eine zumindest teilweise im Inneren des Federrohres angeordnete, in Längsrichtung des Federrohres relativ zum Federrohr verschiebbare Federstange, und ein Hebelelement, das um eine parallel zur ersten Drehachse orientierte zweite Drehachse drehbar mit der Federstange verbunden ist und auf seiner der Federstange gegenüberliegenden Seite um eine von der ersten Drehachse beabstandete, zur ersten Drehachse parallel orientierte dritte Drehachse drehbar an der Befestigungseinheit angeordnet ist.

Bei der vorgeschlagenen Federarmvorrichtung ist das Hebelelement bezogen auf die Längsrichtung des Federrohres radial außerhalb des Federrohres angeordnet. Dadurch ist der Schwenkbereich des Federrohres um die erste Drehachse, im Gegensatz zu Federarmvorrichtungen gemäß dem Stand der Technik, bei welchen das Hebelelement sich in das Innere des Federrohres erstreckt und dort mit der Federstange verbunden ist und der Bewegungsumgang durch die Kollisionspunkte zwischen Hebelelement und der Innenseite des Federrohres begrenzt ist, in seinem Bewegungsumgang nicht eingeschränkt. Vielmehr erlaubt die Anordnung des Hebelelements radial außerhalb des Federrohres, dass das Federrohr und das Hebelelement relativ zu einander im Wesentlichen frei schwenken können, ohne dass dies zu einer Kollision von Hebelelement und Federrohr führt. Der mögliche Schwenkwinkel des Federrohres um die erste Drehachse ist mithin im Wesentlichen lediglich durch die Anbindung des Federrohres an die Befestigungseinheit, nicht aber durch das Hebelelement eingeschränkt. Entsprechend weist die erfindungsgemäße Federarmvorrichtung im Vergleich zu herkömmlichen Federarmvorrichtungen gemäß dem Stand der Technik einen stark vergrößerten Schwenkbereich um die erste Drehachse auf.

Vorzugsweise sind die Federstange und das Hebelelement über ein Verbindungsteil miteinander verbunden. Das Verbindungsteil ist bevorzugt radial innerhalb des Federrohres, mithin im Allgemeinen im Inneren des Federrohres, mit der Federstange verbunden und radial außerhalb des Federrohres mit dem Hebelelement verbunden. Das Verbindungsteil erstreckt sich bevorzugt in Richtung der zweiten Drehachse und/oder bevorzugt durch eine im Federrohr vorgesehenen Aussparung vom Inneren des Federrohres nach radial außerhalb des Federrohres.

Unter "radial innerhalb" wird vorliegend jener Bereich verstanden, welcher in Bezug auf die Längsrichtung des Federrohres eine Erstreckung quer zur Längsrichtung aufweist, die kleiner gleich dem Innenradius des Federrohres bzw. dessen Innenfläche bei unrunden Federrohren ist.

Der Begriff "radial außerhalb" beschreibt jeden Bereich, welcher in Bezug auf die Längsrichtung des Federrohres eine Erstreckung quer zur Längsrichtung aufweist, die größer als der Außenradius des Federrohres bzw. dessen Außenfläche bei unrunden Federrohren ist.

Es hat sich als vorteilhaft herausgestellt, wenn die Aussparung ein in Längsrichtung orientiertes Langloch ist, wobei das Verbindungsteil entlang des Langlochs in Längsrichtung bewegbar ist.

Gemäß einer bevorzugten Ausführungsform weist das Hebelelement in Richtung der ersten Drehachse einen vorgegebenen Abstand zum Federrohr, insbesondere zu einer Anbindung bzw. einem Anbindungsbereich des Federrohres an die Befestigungseinheit, auf. So kann sichergestellt werden, dass das Hebelelement und das Federrohr bei einem Schwenken des Federrohres um die erste Drehachse nicht kollidieren.

Wenn die Federstange als Hohlstange ausgebildet ist, kann durch das Innere der Hohlstange zumindest ein Versorgungskanal, beispielsweise eine Medienversorgung, etwa Druckluft, Sauerstoff oder Stickstoff, und/oder eine Strom- und/oder Datenversorgung, für ein an der Federarmvorrichtung angebrachtes medizinischen Gerät und/oder ein Beleuchtungsgerät angeordnet sein.

Gemäß einer bevorzugten Ausführungsform umfasst die Befestigungseinheit einen Gelenkabschnitt, in welchem das Federrohr und das Hebelelement an die Befestigungseinheit angebunden sind, und ferner einen bevorzugt als Zapfen ausgebildeten Anbindungsabschnitt zum Anbinden der Befestigungseinheit an eine tragende Struktur, bevorzugt eine Decke, eine Wand oder einen Boden eines Raumes, oder einen Tragrahmen oder Tragarm, wobei der Gelenkabschnitt und der Anbindungsabschnitt in Richtung der ersten Drehachse einen vorgegebenen Abstand zueinander aufweisen. Durch diese räumliche Trennung bzw. Beabstandung von Anbindungsabschnitt und Gelenkabschnitt kann die Federarmvorrichtung und insbesondere ein Gehäuse zum Einhausen zumindest des Gelenkabschnitts einen vergleichsweise einfachen Aufbau aufweisen.

Vorzugsweise ist ein bevorzugt drehfest mit dem Federrohr verbundenes erstes Gehäuseteil vorgesehen, welches den Gelenkabschnitt einhaust. Zudem ist vorzugweise ein bevorzugt drehfest an dem Anbindungsabschnitt angebundenes zweites Gehäuseteil vorgesehen, welches vorzugsweise zumindest einen Teil des Anbindungsabschnitts einhaust. Eine Trennebene zwischen dem ersten Gehäuseteil und dem zweiten Gehäuseteil kann senkrecht zur ersten Drehachse orientiert und - in Richtung der ersten Drehachse gesehen - in einem Bereich zwischen dem Gelenkabschnitt und dem Anbindungsabschnitt angeordnet sein.

Auf Höhe dieser Trennebene kann eine Dichtung zum Abdichten des ersten Gehäuseteils und des zweiten Gehäuseteils gegen die Umgebung angeordnet sein.

Gemäß einer weiteren bevorzugten Ausführungsform ist die Federstange im Federrohr translatorisch gelagert, bevorzugt gleitgelagert, wobei bevorzugt die Federstange zumindest einen gegenüber dem Federrohr translatorisch gelagerten, bevorzugt gleitgelagerten, Schlitten umfasst. Bevorzugt umfasst die Federstange an einer ersten Seite einen gegenüber dem Federrohr translatorisch gelagerten ersten Schlitten und an einer zweiten Seite einen gegenüber dem Federrohr translatorisch gelagerten zweiten Schlitten.

Die oben gestellte Aufgabe wird weiterhin durch ein Tragarmsystem zum Halten zumindest eines medizinischen Geräts und/oder eines Beleuchtungsgeräts mit den Merkmalen des Anspruchs 9 gelöst. Vorteilhafte Weiterbildungen ergeben sich aus der vorliegenden Beschreibung und den Figuren.

Entsprechend wird ein Tragarmsystem zum Halten zumindest eines medizinischen Geräts und/oder eines Beleuchtungsgeräts vorgeschlagen, welches zumindest eine Federarmvorrichtung gemäß einer der vorstehenden Ausführungsformen umfasst.

Die hinsichtlich der Federarmvorrichtung beschriebenen Vorteile und Wirkungen werden ebenfalls durch das Tragarmsystem erzielt, weshalb auf deren wiederholte Beschreibung an dieser Stelle verzichtet wird, um Redundanzen zu vermeiden.

### Kurze Beschreibung der Figuren

Bevorzugte weitere Ausführungsformen der Erfindung werden durch die nachfolgende Beschreibung der Figuren näher erläutert. Dabei zeigen:
Figur 1 schematisch eine perspektivische Seitenansicht eines Tragarmsystems mit einer Federarmvorrichtung;
Figur 2 schematisch eine weitere perspektivische Seitenansicht der Federarmvorrichtung aus Figur 1;
Figur 3 schematisch eine perspektivische Seitenansicht eines Teilbereichs des Tragarmsystems aus Figur 1;
Figur 4 schematisch eine weitere perspektivische Seitenansicht des Teilbereichs aus Figur 3;
Figur 5 schematisch eine Darstellung eines Schwenkbereichs eines Federrohres der Federarmvorrichtung um eine erste Drehachse; und
Figur 6 schematische eine perspektivische Seitenansicht der Federarmvorrichtung aus Figur 1 mit einer Punktewolke eines Haltebereichs des Federrohres zum Halten eines Geräts.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

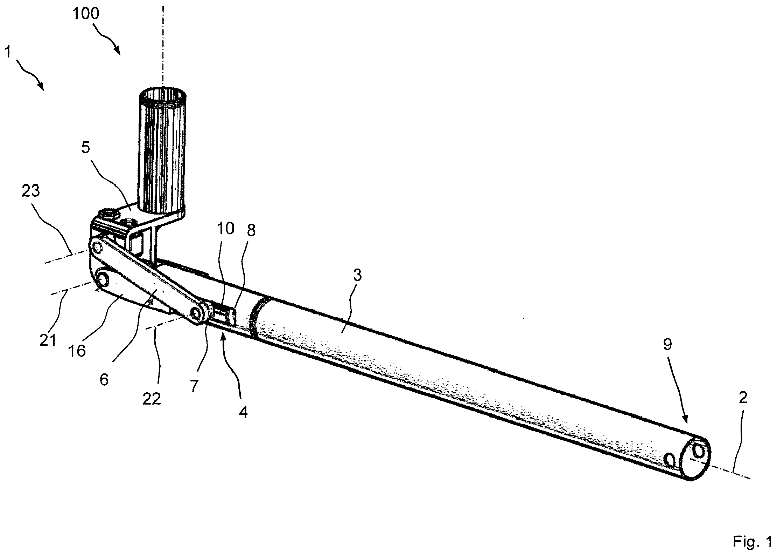

In Figur 1 ist schematisch eine perspektivische Seitenansicht eines Tragarmsystems 100 mit einer Federarmvorrichtung 1 gezeigt. Das Tragarmsystem 100 ist zum Halten eines medizinischen Geräts (nicht gezeigt) oder alternativ eines Beleuchtungsgeräts ausgebildet.

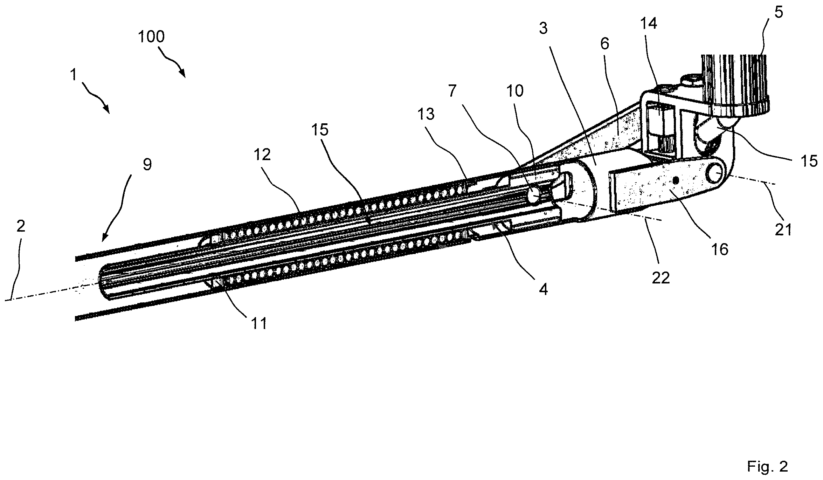

Die Federarmvorrichtung 1 umfasst ein sich in einer Längsrichtung 2 erstreckendes Federrohr 3. im Inneren des Federrohrs 3 ist eine in Längsrichtung 2 relativ zum Federrohr 3 verschiebbare Federstange 4 angeordnet, wie näher in Hinblick auf Figur 2 erläutert. Das Federrohr 3 ist an einer ersten Seite um eine vorliegend in einem Einbauzustand der Federarmvorrichtung am Einsatzort im Wesentlichen horizontal orientierte erste Drehachse 21 drehbar an einer Befestigungseinheit 5 angeordnet.

Die Federarmvorrichtung 1 umfasst ferner ein Hebelelement 6, das um eine parallel zur ersten Drehachse 21 orientierte zweite Drehachse 22 drehbar mit der Federstange 4 verbunden ist und auf seiner der Federstange 4 gegenüberliegenden Seite um eine von der ersten Drehachse 21 beabstandete, zur ersten Drehachse 21 parallel orientierte dritte Drehachse 23 drehbar an der Befestigungseinheit 5 angeordnet ist.

Wie aus Figur 1 zu erkennen, ist das Hebelelement 6 bezogen auf die Längsrichtung 2 radial außerhalb des Federrohres 3 angeordnet.

Die Federstange 4 und das Hebelelement 6 sind über ein Verbindungsteil 7 miteinander verbunden, welches radial innerhalb, also im Inneren, des Federrohres 3 mit der Federstange 4 verbunden ist und radial außerhalb des Federrohres 3 mit dem Hebelelement 6 verbunden ist. Vorliegend erstreckt sich das Verbindungsteil 7 durch eine im Federrohr 3 vorgesehenen Aussparung 8 in Form eines sich in Längsrichtung 2 erstreckenden Langlochs.

An seinem der Befestigungseinheit 5 gegenüberliegendem Ende weist das Federrohr 3 einen Halteabschnitt 9 in Form einer Anbindung zum Anbinden eines zu haltenden Geräts auf. Unter einem Halten eines Geräts wird vorliegend verstanden, dass an dem Halteabschnitt 9 das zu haltende Gerät angebunden ist, indem die Gewichtskraft des Geräts am Halteabschnitt auf das Federrohr 3 eingeleitet wird. Das "Halten" umfasst hierbei auch, dass zwischen dem Gerät und dem Federrohr ein weiterer Tragarm, beispielsweise ein weiterer Federarm, oder eine Haltevorrichtung angeordnet ist.

Figur 2 zeigt schematisch eine perspektivische Seitenansicht der Federarmvorrichtung aus Figur 1 von einer anderen Blickrichtung, wobei ein Teil des Federrohres 3 freigeschnitten gezeigt ist, um den Blick auf das Innere des Federrohres 3 freizugeben.

Im Inneren des Federrohres 3 erstreckt sich die Federstange 4 in Längsrichtung 2. An ihrem der Befestigungseinheit 5 zugewandten Ende umfasst sie einen ersten Schlitten 10, der in Längsrichtung 2 translatorisch im Federrohr 3 gleitgelagert ist. Im Bereich des ersten Schlittens 10 erstreckt sich das mit der Federstange 4 verbundene Verbindungselement 7.

An ihrer gegenüberliegenden Seite umfasst die Federstange 4 einen gegenüber dem Federrohr 3 translatorisch gleitgelagerten zweiten Schlitten 11.

Der zweite Schlitten 11 stellt auf der der Befestigungseinheit 5 gegenüberliegenden Seite der Federstange 4 einen Anschlag für ein Federelement 12, vorliegend ausgebildet als Druckfeder, zum Vorspannen der Federstange 4 relativ zum Federrohr 3 dar. Das Federelement 12 ist auf der anderen Seite an einer Einbuchtung 13 im Federrohr 3 abgestützt. Der Abstand in Längsrichtung 2 zwischen dem zweiten Schlitten 11 und der Einbuchtung 13 ist kleiner als eine Nulllänge des Federelements 12, welches dieses in einem entspannten Zustand aufweist. Das Federelement 12 ist mithin vorgespannt.

Die Höhe der Vorspannung kann über eine Justiereinheit 14 an der Befestigungseinheit 5 angepasst werden. Die Justiereinheit 14 ermöglicht es, den Abstand der dritten Drehachse 23 relativ zur ersten Drehachse 21 zu verändern. Die Vorspannung ist im Wesentlichen abhängig von der Hebellänge, also dem Abstand zwischen der ersten Drehachse 21 und der dritten Drehachse 23.

Mit dem Bezugszeichen 15 ist ein Stromkabel bezeichnet, das sich von der Befestigungseinheit 5 kommend durch die als hohle Stange ausgebildete Federstange 4 zum Halteabschnitt 9 erstreckt.

Wie in Zusammenschau der Figuren 1 und 2 zu erkennen, umfasst das Federrohr 3 einen gabelförmigen Anbindungsbereich 16 zum Anbinden an die Befestigungseinheit 5.

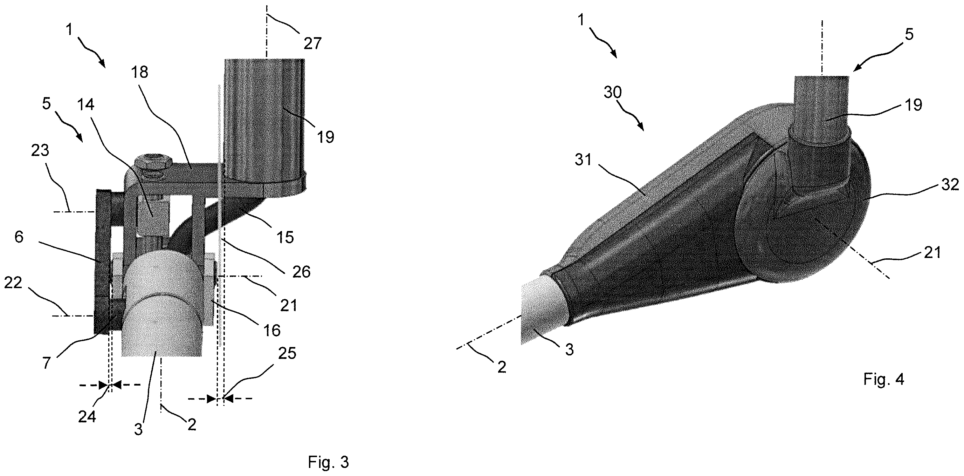

Figur 3 zeigt schematisch eine Seitenansicht auf einen Teilbereich der Federarmvorrichtung 1 aus Figur 1.

Aus dieser Ansicht ist zu erkennen, dass das Hebelelement 6 in Richtung der ersten Drehachse 21 einen vorgegebenen Abstand 24 zum Federrohr 3, genauer zur Anbindung des Federrohres 3 an die Befestigungseinheit 5, aufweist. Entsprechend ist ermöglicht, dass das Hebelelement 6 und das Federrohr 3 sich aneinander vorbei bewegen können, ohne zu kollidieren.

Die Befestigungseinheit 5 ist vorliegend in einen Gelenkabschnitt 18, in welchem das Federrohr 3 und das Hebelelement 6 an die Befestigungseinheit 5 angebunden sind, und einen mit dem Gelenkabschnitt 18 verbundenen, als Zapfen ausgebildeten Anbindungsabschnitt 19 zum Anbinden der Befestigungseinheit 5 an eine tragende Struktur, beispielsweise eine Decke, eine Wand oder einen Boden eines Raumes, oder einen Tragrahmen oder Tragarm, unterteilt.

Der Gelenkabschnitt 18 und der Anbindungsabschnitt 19 weisen in Richtung der ersten Drehachse 21 betrachtet einen vorgegebenen Abstand 25 zueinander auf. Dies ermöglicht es, eine senkrecht zur ersten Drehachse 21 orientierte Trennebene 26 für ein zweiteiliges Gehäuse (siehe Figur 4) - in Richtung der ersten Drehachse 21 gesehen - zwischen Gelenkabschnitt 18 und Anbindungsabschnitt 19 vorzusehen.

Aus Figur 4 ist schematisch eine perspektivische Seitenansicht auf den Teilbereich aus Figur 3 zu entnehmen, wobei hier ein zweitteiliges Gehäuse 30 mit dargestellt ist.

Das Gehäuse 30 haust den Gelenkabschnitt 18 sowie die Durchführung des Stromkabels 15 im Bereich des Anbindungsabschnitts 19 gegen die Umgebung ein.

Das Gehäuse 30 umfasst ein drehfest mit dem Federrohr 3 verbundenes erstes Gehäuseteil 31, welches den Gelenkabschnitt einhaust, und ein drehfest mit dem Anbindungsabschnitt 19 verbundenes zweites Gehäuseteil 32, welches einen unteren Teil des Anbindungsabschnitts 19 einhaust. Das erste Gehäuseteil 31 ist relativ zum zweiten Gehäuseteil 32 um die erste Drehachse 21 schwenkbar, wobei das erste Gehäuseteil den Bewegungen des Federrohres 3 folgt.

Wie bereits zu Figur 3 beschrieben, liegt die Trennebene 26 zwischen dem ersten Gehäuseteil 31 und dem zweiten Gehäuseteil 32 in Richtung der ersten Drehachse 21 gesehen in einem Bereich zwischen dem Gelenkabschnitt 18 und dem Anbindungsabschnitt 19. Auf Höhe der Trennebene 26 ist eine Dichtung zum Abdichten des ersten Gehäuseteils 31 und des zweiten Gehäuseteils 32 gegen die Umgebung angeordnet. Die Dichtung ist vorliegend ausgebildet als eine Welle-Nabe-Verbindung mit Spielpassung zwischen den Gehäuseteilen 31, 32. Das zweite Gehäuseteil 32 umfasst einen in Figur 4 nicht sichtbaren, sich um die erste Drehachse 21 kreiszylindrisch erstreckenden Zapfenabschnitt, welcher in einem sich um die erste Drehachse 21 kreiszylindrisch erstreckenden Nabenabschnitt aufgenommen ist. Die radiale Außenfläche des Zapfenabschnitts und die radiale Innenfläche des Nabenabschnitts weisen eine vorgegebene Spielpassung auf.

Alternativ oder zusätzlich kann die Dichtung auch eine Labyrinthdichtung und/oder ein Dichtelement, beispielsweise einen Dichtring, umfassen.

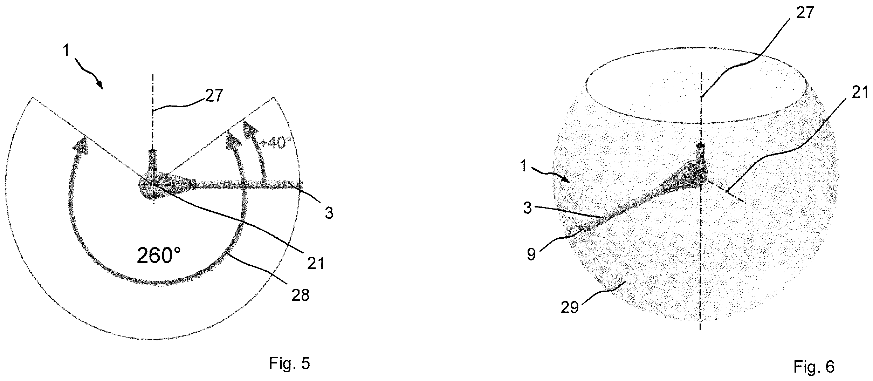

Figur 5 zeigt schematisch eine Darstellung eines Schwenkbereichs des Federrohres 3 der Federarmvorrichtung 1 aus Figur 1 um die erste Drehachse 21. Die erste Drehachse 21 ist vorliegend im Wesentlichen horizontal orientiert. Wie aus Figur 5 zu erkennen, umfasst das Federrohr 3 aus einer horizontalen Stellung heraus wie in Figur 5 gezeigt einen Schwenkbereich von 40° gegen die Erdbeschleunigung, also nach oben. Das Federrohr 3 kann unten herum über die Vertikale schwenken, so dass der Schwenkbereich aus der Horizontalen gegen die Erdbeschleunigung auf der anderen Seite ebenfalls bei 40° liegt.

Insgesamt ergibt sich bei der Federarmvorrichtung 1 ein Schwenkwinkel 28 des Federrohrs 3 um die erste Drehachse 21 von optional 260°.

Die vorstehend genannten Schwenkbereiche von 40° und 260° sind lediglich beispielhaft und die Federarmvorrichtung ist nicht auf diese Schwenkbereiche beschränkt. Sie können je nach Anwendungsfall und Bedarf andere Werte aufweisen. Auch kann der Schwenkbereich gegenüber der Vertikalen auf einer Seite unterschiedlich zur anderen Seite ausgebildet sein.

Figur 6 zeigt schematische eine perspektivische Seitenansicht der Federarmvorrichtung 1 aus Figur 5 mit einer Punktewolke 29 des Haltebereichs 9 des Federrohres 3. Die Federarmvorrichtung 1 ist um eine vertikale Drehachse 27 herum schwenkbar ausgebildet. Entsprechend ergibt sich die Punktewolke 29, also die Summe aller durch den Haltebereich 9 aufgrund des Bewegungsumfangs der Federarmvorrichtung 1 erreichbaren Punkte, ein Kugelsegment, welche sich zusammensetzt aus dem Schwenkbereich aus Figur 5, rotiert um die vertikale Drehachse 27.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Federarmvorrichtung
- 2: Längsrichtung
- 3: Federrohr
- 4: Federstange
- 5: Befestigungseinheit
- 6: Hebelelement
- 7: Verbindungsteil
- 8: Aussparung
- 9: Halteabschnitt
- 10: Erster Schlitten
- 11: Zweiter Schlitten
- 12: Federelement
- 13: Einbuchtung
- 14: Justiereinheit
- 15: Stromkabel
- 16: Anbindungsbereich
- 18: Gelenkabschnitt
- 19: Anbindungsabschnitt
- 21: Erste Drehachse
- 22: Zweite Drehachse
- 23: Dritte Drehachse
- 24: Abstand
- 25: Abstand
- 26: Trennebene
- 27: Drehachse
- 28: Schwenkwinkel
- 29: Punktewolke
- 30: Gehäuse
- 31: Erstes Gehäuseteil
- 32: Zweites Gehäuseteil
- 100: Tragarmsystem

## Patentansprüche

1. Federarmvorrichtung (1) für ein Tragarmsystem (100) zum Halten eines medizinischen Geräts und/oder eines Beleuchtungsgeräts, umfassend ein um eine erste Drehachse (21) drehbar an einer Befestigungseinheit (5) angeordnetes Federrohr (3), eine zumindest teilweise im Inneren des Federrohres (3) angeordnete, in Längsrichtung (2) des Federrohres (3) relativ zum Federrohr (3) verschiebbare Federstange (4), und ein Hebelelement (6), das um eine parallel zur ersten Drehachse (21) orientierte zweite Drehachse (22) drehbar mit der Federstange (4) verbunden ist und auf seiner der Federstange (4) gegenüberliegenden Seite um eine von der ersten Drehachse (21) beabstandete, zur ersten Drehachse (21) parallel orientierte dritte Drehachse (23) drehbar an der Befestigungseinheit (5) angeordnet ist,
**dadurch gekennzeichnet, dass**
das Hebelelement (6) bezogen auf die Längsrichtung (2) des Federrohres (3) radial außerhalb des Federrohres (3) angeordnet ist.

2. Federarmvorrichtung (1) gemäß Anspruch 1, **dadurch gekennzeichnet, dass** die Federstange (4) und das Hebelelement (6) über ein Verbindungsteil (7) miteinander verbunden sind, wobei das Verbindungsteil (7) radial innerhalb des Federrohres (3) mit der Federstange (4) verbunden ist und radial außerhalb des Federrohres (3) mit dem Hebelelement (6) verbunden ist.

3. Federarmvorrichtung (1) gemäß Anspruch 2, **dadurch gekennzeichnet, dass** sich das Verbindungsteil (7) durch eine im Federrohr (3) vorgesehenen Aussparung (8) erstreckt.

4. Federarmvorrichtung (1) gemäß Anspruch 3, **dadurch gekennzeichnet, dass** die Aussparung (8) ein in Längsrichtung (2) orientiertes Langloch ist, wobei das Verbindungsteil (7) entlang des Langlochs in Längsrichtung (2) bewegbar ist.

5. Federarmvorrichtung (1) gemäß einem der vorstehenden Ansprüche, **dadurch gekennzeichnet, dass** das Hebelelement (6) in Richtung der ersten Drehachse (21) einen vorgegebenen Abstand (24) zum Federrohr (3), insbesondere zu einem Anbindungsbereich (16) des Federrohres (3) an die Befestigungseinheit (5), aufweist.

6. Federarmvorrichtung (1) gemäß einem der vorstehenden Ansprüche, **dadurch gekennzeichnet, dass** die Federstange (4) als Hohlstange ausgebildet ist.

7. Federarmvorrichtung (1) gemäß einem der vorstehenden Ansprüche, **dadurch gekennzeichnet, dass** die Befestigungseinheit (5) einen Gelenkabschnitt (18) umfasst, in welchem das Federrohr (3) und das Hebelelement (6) an die Befestigungseinheit (5) angebunden sind, und einen Anbindungsabschnitt (19) zum Anbinden der Befestigungseinheit (5) an eine tragende Struktur, bevorzugt eine Decke, eine Wand oder einen Boden eines Raumes, oder einen Tragrahmen oder Tragarm umfasst, wobei der Gelenkabschnitt (18) und der Anbindungsabschnitt (19) in Richtung der ersten Drehachse (21) einen vorgegebenen Abstand (25) zueinander aufweisen.

8. Federarmvorrichtung (1) gemäß dem vorstehenden Anspruch, **dadurch gekennzeichnet, dass** der Anbindungsabschnitt (19) als Zapfen ausgebildet ist.

9. Federarmvorrichtung (1) gemäß Anspruch 7 oder 8, **dadurch gekennzeichnet, dass** ein mit dem Federrohr (3) verbundenes erstes Gehäuseteil (31) den Gelenkabschnitt (18) einhaust und ein mit dem Anbindungsabschnitt (19) verbundenes zweites Gehäuseteil (32) zumindest einen Teil des Anbindungsabschnitts (19) einhaust, wobei eine Trennebene (26) zwischen dem ersten Gehäuseteil (31) und dem zweiten Gehäuseteil (32) in Richtung der ersten Drehachse (21) gesehen in einem Bereich zwischen dem Gelenkabschnitt (18) und dem Anbindungsabschnitt (19) senkrecht zur ersten Drehachse (21) angeordnet ist.

10. Federarmvorrichtung (1) gemäß dem vorstehenden Anspruch, **dadurch gekennzeichnet, dass** auf Höhe der Trenneben (26) eine Dichtung zum Abdichten des ersten Gehäuseteils (31) und des zweiten Gehäuseteils (32) gegen die Umgebung angeordnet ist.

11. Federarmvorrichtung (1) gemäß Anspruch 9 oder 10, **dadurch gekennzeichnet, dass** das erste Gehäuseteil (31) drehfest mit dem Federrohr (3) verbunden ist, und/oder das zweite Gehäuseteil (32) drehfest mit dem Anbindungsabschnitt (19) verbunden ist.

12. Federarmvorrichtung (1) gemäß einem der vorstehenden Ansprüche, **dadurch gekennzeichnet**, die Federstange (4) im Federrohr (3) translatorisch gelagert, bevorzugt gleitgelagert, ist.

13. Federarmvorrichtung (1) gemäß dem vorstehenden Anspruch, **dadurch gekennzeichnet, dass** die Federstange (4) zumindest einen gegenüber dem Federrohr (3) translatorisch gelagerten, bevorzugt gleitgelagerten, Schlitten (10, 11) umfasst.

14. Federarmvorrichtung (1) gemäß Anspruch 12 oder 13, **dadurch gekennzeichnet, dass** die Federstange (4) an einer ersten Seite einen gegenüber dem Federrohr (3) translatorisch gelagerten ersten Schlitten (10) umfasst und an einer zweiten Seite einen gegenüber dem Federrohr (3) translatorisch gelagerten zweiten Schlitten (11) umfasst.

15. Tragarmsystem (100) zum Halten zumindest eines medizinischen Geräts und/oder eines Beleuchtungsgeräts, umfassend zumindest eine Federarmvorrichtung (1) gemäß einem der vorstehenden Ansprüche.
